# EUROPEAN PATENT SPECIFICATION

(11) **EP 3 797 099 B1**
(45) Date of publication and mention of the grant of the patent: **01.11.2023**
(21) Application number: 19734512.7
(22) Date of filing: 20.05.2019
(51) Int. Cl.: C07D 231/14

(54) **METHOD FOR PREPARING SUBSTITUTED HETEROCYCLIC COMPOUNDS**
VERFAHREN ZUR HERSTELLUNG VON SUBSTITUIERTEN HETEROCYCLISCHEN VERBINDUNGEN
PROCÉDÉ DE PRÉPARATION DE COMPOSÉS HÉTÉROCYCLIQUES SUBSTITUÉS

(30) Priority: 21.05.2018 IN 201811018994
(43) Date of publication of application: 31.03.2021
(73) Proprietor: PI Industries Ltd., Udaipur, Rajasthan 313001 (IN)
(72) Inventor: SYTHANA, Suresh Kumar, Pradesh 500048 (IN); SALVI, Vijay Kumar, Udaipur-Raiasthan 313001 (IN); PANDYA, Mayank, Dungarpur-Rajasthan 314402 (IN); KANAWADE, Shrikant Bhausaheb, Nashik-Maharashtra 422010 (IN); KLAUSENER, Alexander G. M., 50259 Pulheim (DE)
(74) Representative: Keltie LLP
(86) International application number: PCT/IB2019/054119
(87) International publication number: WO 2019/224677

(56) References cited:
- EP-A1- 2 552 895
- WO-A1-03/051820
- WO-A1-2009/112157
- WO-A1-2009/133178
- WO-A2-2008/022777
- MAMTA SURI ET AL: "An efficient copper-catalyzed formation of highly substituted pyrazoles using molecular oxygen as the oxidant", GREEN CHEMI, ROYAL SOCIETY OF CHEMISTRY, GB, vol. 14, 1 January 2012 (2012-01-01), pages 2193-2196, XP009172870, ISSN: 1463-9262, DOI: 10.1039/C2GC35476D

## Description

### Field of the Invention

The present invention relates to a novel process as defined in the claims for the preparation of substituted heterocyclic compound of Formula I or of salts thereof.

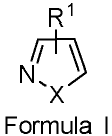

### Background of the Invention

Substituted heterocyclic compounds of Formula I or of salts thereof are important starting materials for a number of active pharmaceutical ingredients and crop protection active ingredients.

Processes for the preparation of substituted pyrazole compounds are described widely in literature, e.g. WO2003051820; WO2008102678; WO2008145257; WO200853043; WO200900442; WO2008022777; WO2009133178; WO2009133179; WO201009990; WO201154732; WO2012163905; WO2015003289; WO2015063793; WO2016152831; CN102718712; CN102731402; CN102766096; CN103351339; CN103360313; CN103787977; CN104016920; CN104163800; CN104326891; CN104326891; CN105218448; EP1997808; GB200908435; JP2013006780 and Angew. Chem. Int. Ed. 2010, 49, 7790 -7794.

The processes described in the prior art though provide moderate to good yields, however, result in a mixture of regioisomeric pyrazoles. Another drawback of these processes is the use of reactants as methylhydrazine or substituted hydrazines, which are highly carcinogenic in nature and are difficult to handle. Moreover, the existing methodologies have a higher number of reaction steps, thereby making it unfavourable to run them on a commercial scale.

WO2009112157 discloses regioselective synthesis of 1-alkyl-3-halo-alkylpyrazole-4-carboxylic acid derivatives by cyclization of 2,3-disubstituted acrylic acid derivatives with hydrazines in the presence of carbonyl compounds. However, it has been noted that the reaction uses substituted hydrazines, which are highly carcinogenic in nature and are difficult to handle.

Article titled "An efficient copper-catalyzed formation of highly substituted pyrazoles using molecular oxygen as the oxidant" by Mamta Suri et Al, published in Green Chem., 2012, 14, 2193-2196 discloses formation of highly substituted pyrazoles by reacting enamines and nitriles. However, it has been noted that an additive is an essential component to facilitate the reactions to obtain the desired product. Further, reactions are carried out at higher temperatures.

EP2552895 discloses pyrazole synthesis by coupling of carboxylic acid derivatives and enamines. However, it has been noted that the nitrile reactant is used as a solvent and reactions are carried out at higher temperatures.

Therefore, there is a need for a simple and regioselective process for the synthesis of substituted pyrazoles. Accordingly, the present invention provides a regioselective, industrially feasible, and cost-effective process for the preparation of a substituted heterocyclic compound of Formula I.

### Objective and Summary of the Invention:

It is an objective of the present invention to provide a simple, regioselective, industrially feasible, safe and cost-effective process for the preparation of substituted heterocyclic compounds of Formula I or of salts thereof.

Surprisingly, the present invention provides a solution to these objectives by offering a novel high yielding and economically attractive process overcoming at least one of the shortcomings of the processes described in the prior art.

The objective was achieved according to the present invention by finding a novel process for preparing substituted heterocyclic compounds of Formula I or of salts thereof 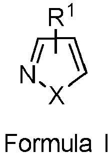
wherein **R¹** is selected from hydrogen, halogen, COOR³, C₁-C₆-alkyl, C₂-C₆-alkenyl, C₃-C₈-cyclohaloalkyl, C₁-C₆-haloalkyl, C₁-C₆-haloalkoxy, C₁-C₆-alkoxy-C₁-C₄-alkyl, C₃-C₈-cycloalkoxy, C₁-C₆-hydroxyalkyl, C₃-C₈-halocycloalkyl, C₁-C₆-haloalkoxycarbonyl, C₁-C₆-alkylthio, C₁-C₆-alkylsulfinyl, C₁-C₆-alkylsulfonyl, C₁-C₆-haloalkylthio, C₁-C₆-haloalkylsulfinyl, C₁-C₆-haloalkylsulfonyl, C₃-C₈-cycloalkylthio, C₁-C₆-alkylamino, C₁-C₆-dialkylamino, C₃-C₈-cycloalkylamino, C₃-C₈-cycloalkyl-C₁-C₆-alkylamino, C₁-C₆-alkoxycarbonyl, C₁-C₆-alkoxycarbonyloxy, C₁-C₆-alkylaminocarbonyloxy, C₁-C₆-dialkylaminocarbonyloxy, 5- to 11-membered spirocyclic ring or 3- to 10- membered carbocyclic ring; wherein Formula I may be substituted by one or more R¹;
**X** is NR²;
**R²** is C₁-C₆-alkyl;
**R³** is C₁-C₆-alkyl;
which comprises reacting a compound of Formula II or of salts thereof R¹ 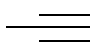 N Formula II
wherein R¹ has the same meaning as defined above;
with a compound of Formula III or of salts thereof 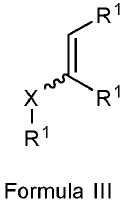
wherein X and R¹ have the same meaning as defined above;
in the presence of a catalyst and optionally in the presence of a solvent selected from one or more of aliphatic, alicyclic or aromatic hydrocarbons and ethers; wherein said reaction is carried out at temperature in the range of 50°C to 95°C.

### Detailed Description of the Invention:

The definitions provided herein for the terminologies used in the present disclosure are for illustrative purpose only and in no manner limit the scope of the present invention disclosed in the present disclosure.

As used herein, the terms "comprises", "comprising", "includes", "including", "has", "having", "contains", "containing", "characterized by" or any other variation thereof, are intended to cover a non-exclusive inclusion, subject to any limitation explicitly indicated. For example, a composition, mixture, process or method that comprises a list of elements is not necessarily limited to only those elements but may include other elements not expressly listed or inherent to such composition, mixture, process or method.

The transitional phrase "consisting of" excludes any element, step or ingredient not specified. If in the claim, such would close the claim to the inclusion of materials other than those recited except for impurities ordinarily associated therewith. When the phrase "consisting of" appears in a clause of the body of a claim, rather than immediately following the preamble, it limits only the element set forth in that clause; other elements are not excluded from the claim as a whole.

The transitional phrase "consisting essentially of" is used to define a composition or method that includes materials, steps, features, components or elements, in addition to those literally disclosed, provided that these additional materials, steps, features, components or elements do not materially affect the basic and novel characteristic(s) of the claimed invention. The term "consisting essentially of" occupies a middle ground between "comprising" and "consisting of".

Further, unless expressly stated to the contrary, "or" refers to an inclusive "or" and not to an exclusive "or". For example, a condition A "or" B is satisfied by any one of the following: A is true (or present) and B is false (or not present), A is false (or not present) and B is true (or present), and both A and B is true (or present).

Carbon-based radical refers to a monovalent molecular component comprising a carbon atom that connects the radical to the remainder of the chemical structure through a single bond. Carbon-based radicals can optionally comprise saturated, unsaturated and aromatic groups, chains, rings and ring systems, and heteroatoms. Although carbon-based radicals are not subject to any particular limit in size, in the context of the present invention they typically comprise 1 to 16 carbon atoms and 0 to 3 heteroatoms. Of note are carbon-based radicals selected from C₁-C₆ alkyl, C₁-C₆ haloalkyl and phenyl optionally substituted with 1-3 substituents selected from C₁-C₃ alkyl, halogen and nitro.

The meaning of various terms used in the description shall now be illustrated.

The term "alkyl", used either alone or in compound words such as "alkylthio" or "haloalkyl" or - N(alkyl) or alkylcarbonylalkyl or alkylsulphonylamino includes straight-chain or branched C₁ to C₂₄ alkyl, preferably C₁ to C₁₅ alkyl, more preferably C₁ to C₁₀ alkyl, most preferably C₁ to C₆ alkyl. Representative examples of alkyl include methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl or the different isomers. If the alkyl is at the end of a composite substituent, as, for example, in alkylcycloalkyl, the part of the composite substituent at the start, for example the cycloalkyl, may be mono- or polysubstituted identically or differently and independently by alkyl. The same also applies to composite substituents in which other radicals, for example alkenyl, alkynyl, hydroxyl, halogen, carbonyl, carbonyloxy and the like, are at the end.

The term "cycloalkyl" means alkyl closed to form a ring. Representative examples include but are not limited to cyclopropyl, cyclobutyl, cyclopentyl and cyclohexyl. This definition also applies to cycloalkyl as a part of a composite substituent, for example cycloalkylalkyl etc., unless specifically defined elsewhere.

The term "cycloalkylalkyl" means cycloalkyl substituent on alkyl, for example, cyclopropyl or cyclobutyl or cyclopentyl are substituted on any carbon of C₁-C₆ alkyl. Representative examples of cycloalkylalkyl include cyclopropyl methyl, cyclopropyl ethyl.

As used herein, the term "combined" refers to the act of "mixing", "intermixing" or "putting together" for the purposes of bringing two or more chemical compounds in close contact so as to promote a chemical reaction. For example certain substrates, reagents or ingredients, reagents as described in the summary of the invention are "combined" with each other in an appropriate vessel, container or apparatus in such a fashion that the substrates, reagents or ingredients can chemically react with one another so that a new product can be formed.

The present invention relates to a novel process for the preparation of substituted heterocyclic compounds of Formula I or of salts thereof 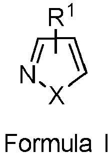
wherein **R¹** is selected from hydrogen, halogen, COOR³, C₁-C₆-alkyl, C₂-C₆-alkenyl, C₃-C₈-cyclohaloalkyl, C₁-C₆-haloalkyl, C₁-C₆-haloalkoxy, C₁-C₆-alkoxy-C₁-C₄-alkyl, C₃-C₈-cycloalkoxy, C₁-C₆-hydroxyalkyl, C₃-C₈-halocycloalkyl, C₁-C₆-haloalkoxycarbonyl, C₁-C₆-alkylthio, C₁-C₆-alkylsulfinyl, C₁-C₆-alkylsulfonyl, C₁-C₆-haloalkylthio, C₁-C₆-haloalkylsulfinyl, C₁-C₆-haloalkylsulfonyl, C₃-C₈-cycloalkylthio, C₁-C₆-alkylamino, C₁-C₆-dialkylamino, C₃-C₈-cycloalkylamino, C₃-C₈-cycloalkyl-C₁-C₆-alkylamino, C₁-C₆-alkoxycarbonyl, C₁-C₆-alkoxycarbonyloxy, C₁-C₆-alkylaminocarbonyloxy, C₁-C₆-dialkylaminocarbonyloxy, 5- to 11-membered spirocyclic ring or 3- to 10- membered carbocyclic ring; wherein Formula I may be substituted by one or more R¹;
**X** is NR²;
**R²** is C₁-C₆-alkyl;
**R³** is C₁-C₆-alkyl;
which comprises reacting a compound of Formula II or of salts thereof R 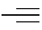 N
   Formula II
wherein R¹ has the same meaning as defined above;
with a compound of Formula III or of salts thereof 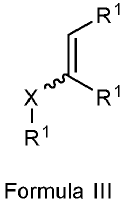
wherein X and R¹ have the same meaning as defined above;
in the presence of a catalyst and optionally in the presence of a solvent selected from one or more of aliphatic, alicyclic or aromatic hydrocarbons and ethers; wherein said reaction is carried out at temperature in the range of 50°C to 95°C.

The catalyst is selected from the group comprising of one or more of the species of copper powder, anhydrous copper(II) acetate, copper(II) acetate monohydrate, copper(I) oxide, copper(II) oxide, copper(I) chloride, copper(II) chloride, iron(II) chloride, iron(III) chloride, zinc(II) chloride, nickel(II) acetate, cobalt(II) chloride anhydrous, cobalt(II) chloride hexahydrate, cobalt(II) chloride dihydrate, cobalt(II, III) oxide hexahydrate, cobalt(II) bromide, cobalt(II) fluoride, cobalt(II) iodide, cobalt(II) oxide, cobalt(III) oxide, cobalt(II) acetate anhydrous, cobalt(II) acetate tetrahydrate and the like. The preferred catalyst is selected from copper compounds such as copper oxides and/or copper salts. The catalyst can be utilized in catalytic or stoichiometric amounts either independently or in combinations.

The solvent is selected from the group comprising of aliphatic, alicyclic or aromatic hydrocarbons, ethers; aliphatic hydrocarbons are selected from but not limited to hexane, heptane, octane and the like; alicyclic hydrocarbons like cycloalkanes are selected from but not limited to cyclopentane, cyclohexane, cycloheptane, cyclooctane and the like; aromatic hydrocarbons are selected from but not limited to toluene, xylene, mesitylene, benzene and the like; ethers are selected from but not limited to diethyl ether, diisopropyl ether, t-butyl methyl ether, tetrahydrofuran, 2-methyl tetrahydrofuran, dioxane, monoglyme, diglyme, methoxy-methane, methoxy-ethane, ethoxy-ethane, di-methoxyethane, di-ethoxyethane and the like.

The reaction of a compound of Formula II or of salts thereof with a compound of Formula III or of salts thereof may be carried out in an autoclave oxygen under pressure or autogenous pressure, or atmospheric pressure or reduced pressure. The reaction is preferably carried out in the presence of inert solvents and at reflux temperature.

According to a preferred embodiment, to a solution of a compound of Formula II or of salts thereof, in a suitable solvent, a solution of compound of Formula III or of salts thereof, in a suitable solvent and a catalyst are added. The reaction can be carried out at a temperature between 50 °C to 95 °C, wherein said reaction may be carried out in presence of oxygen/ air, under reduced pressure or at atmospheric pressure or under pressure conditions. The reaction mixture is usually stirred for 16 h to 48 h, to obtain a compound of Formula I or of salts thereof. The stoichiometry of the catalyst used is in the range of 1 mol% to 100 mol%, preferably 10 mol% to 30 mol%. Compounds of Formulae VI, VII, VIII or of salts thereof are undesired products whereas water, acetic acid and acetic anhydride are by-products. These impurities and by-products can be removed by reaction work-up techniques such as acid base treatment or/and crystallization or/and distillation.

Yet another embodiment of the present invention provides a novel process for preparing substituted heterocyclic compound of Formula I or of salts thereof, having a purity of more than 99%, when measured by GC method.

According to the present invention, after completion of the reaction, isolation of the desired product is carried out by using suitable conventional techniques of reaction work-up such as separation/ solvent extraction/ triturating, acid-base wash, chromatography, filtration, sedimentation, centrifugation and/or washing.

A specific embodiment of the present invention provides a novel and efficient synthesis of compounds of Formula I or of salts thereof, having impurities of less than 10%, wherein the impurities are selected from the group comprising of Formulae VI, VII, VIII, IX, and X or of salts thereof.

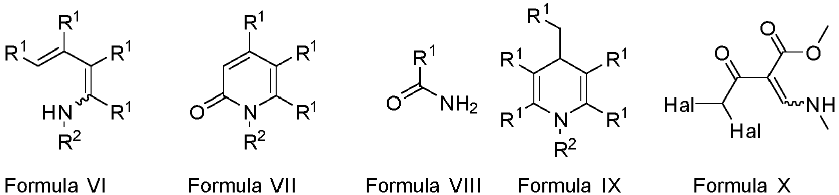

A specific embodiment of the present invention provides a novel and efficient synthesis of compounds of Formula I or of salts thereof, having impurities of less than 10%, wherein the impurities are selected from the group comprising of Formulae VIA, VIIA, VIIIA, IXA, and XA or of salts thereof.

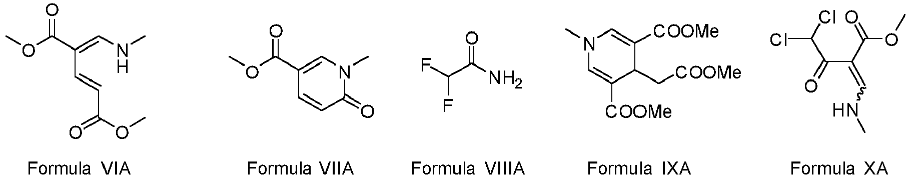

A specific embodiment of the present invention provides a substantially pure methyl-3-(dihalomethyl)-1-methyl-1H-pyrazole-4-carboxylate compound of Formula 1A 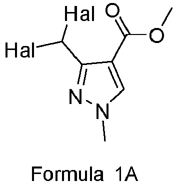 comprising impurities of less than 10%, wherein the impurities are selected from the group comprising of Formulae VI, VII, VIII, IX, and X or of salts thereof.

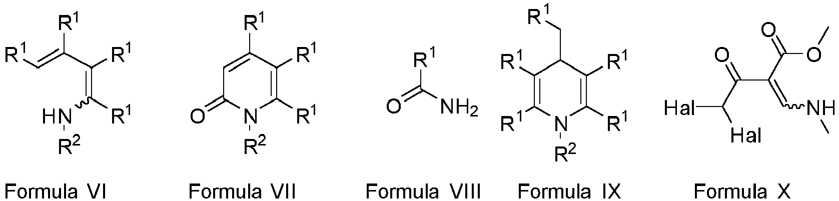

The term "substantially pure" methyl-3-(dihalomethyl)-1-methyl-1H-pyrazole-4-carboxylate of Formula 1A means that any impurities are less than 10%, preferably less than 8%, more preferably less than 5%, as per GC, wherein the impurities are selected from the group comprising of Formulae VI, VII, VIII, IX, and X or of salts thereof.

A specific embodiment of the present invention provides a process for preparing methyl-3-(dihalomethyl)-1-methyl-1H-pyrazole-4-carboxylate compound of Formula 1A 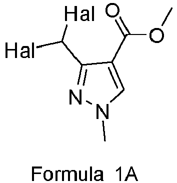 comprising reacting 2,2-dihaloacetonitrile with methyl-3-(methylamino)acrylate in the presence of a catalyst and optionally in the presence of a solvent selected from one or more of aliphatic, alicyclic or aromatic hydrocarbons and ethers.

The present invention is further described in greater detail as illustrated in the following non-limiting examples. It should be understood that variation and modification of the process are possible within the ambit of the invention broadly disclosed herein.

### Examples

### Example-1: Preparation of methyl-3-(difluoromethyl)-1-methyl-1H-pyrazole-4-carboxylate

Copper(I) oxide (1.12 g, 7.82 mmol) and anhydrous copper(II) acetate (0.16 g, 0.869 mmol) were added to a solution of 2,2-difluoroacetonitrile (0.669 g, 8.69 mmol) in toluene (17.3 ml). The reaction mixture was heated to 40 °C for 1 h. A solution ofmethyl-3-(methylamino)acrylate (1.0 g, 9.69 mmol) in toluene (5.76 ml) was slowly added to it. The reaction mixture was stirred further for 16 h at 65-70 °C, cooled to 25-30 °C and then filtered through a celite bed. The filtrate was washed with 10% aqueous citric acid solution (5.0 ml), 10% aqueous sodium bicarbonate solution (5.0 ml). Organic layer was dried over anhydrous sodium sulphate, and concentrated under reduced pressure to obtain methyl-3-(difluoromethyl)-1-methyl-1H-pyrazole-4-carboxylate (0.6 g, 36.3%, 3.16 mmol).

**¹H NMR (CDCl_{3,} 400 MHz):** δ 7.87 (s, 1H), 7.07 (t, *J* = 54 Hz, 1H), 3.94 (s, 3H), 3.82 (s, 3H).

**¹³C NMR (CDCl_{3,} 100 MHz):** δ 39.6, 51.6, 106.8, 109.2, 111.5, 112.9, 134.9, 145.9, 146.2, 146.4, 162.1.

GC-MS: 190.1

### Example-2: Preparation of methyl-3-(difluoromethyl)-1-methyl-1H-pyrazole-4-carboxylate

A 100 ml autoclave was charged with methyl-3-(methylamino)acrylate (1.50 g, 12.38 mmol), 2,2-difluoroacetonitrile (1.00 g, 13.00 mmol), anhydrous copper(II) acetate (0.67 g, 3.71 mmol) and toluene (25.0 ml) at 25 °C. O₂-pressure (1 bar) was applied under vigorous stirring. The reaction mixture was stirred vigorously at 90 °C for 20 h. It was cooled to 25 °C and filtered through a celite bed. The filtrate was washed with 10% aqueous citric acid solution (20.0 ml). The aqueous citric acid layer was washed twice with ethyl acetate (100.0 ml). The combined organic layers were washed with water (20.0 ml), dried over anhydrous sodium sulphate and concentrated under reduced pressure to obtain methyl-3-(difluoromethyl)-1-methyl-1H-pyrazole-4-carboxylate (1.91 g, 81% yield, 10.04 mmol).

### Example-3: Preparation of methyl-3 -(dichloromethyl)-1-methyl-1H-pyrazole-4-carboxylate

To a solution of 2,2-dichloro acetonitrile (23.9 g, 217.0 mmol) in toluene (461 ml) was added anhydrous copper(II) acetate (39.4 g, 217.0 mmol), and mild vacuum (703 Torr) was applied at 25-30 °C. The reaction mixture was heated to 65-70 °C and a solution of methyl-3-(methylamino)acrylate (25.0 g, 217.0 mmol) in toluene (115.0 ml) was added to it over 1 h. After complete addition, the reaction mixture was stirred for 16 h at 65-70 °C under vacuum conditions (703 Torr). The reaction mixture was then cooled to 25-30 °C and filtered through a celite bed. The filtrate was washed with 10% aqueous citric acid solution (416.0 ml), 10% sodium bicarbonate solution (383.0 ml) and water (250.0 ml). The organic layer was dried over anhydrous sodium sulphate and concentrated under reduced pressure to obtain methyl-3-(dichloromethyl)-1-methyl-1H-pyrazole-4-carboxylate (28.0 g, 57.7% yield, 125.53 mmol).

**¹H NMR (CDCl_{3,} 400 MHz):** δ 7.82 (s, 1H), 7.39 (s, 1H), 3.97 (s, 3H), 3.85 (s, 3H).

**¹³C NMR (CDCl_{3,}100 MHz):** δ 39.6, 51.4, 62.3, 109.9, 134.5, 151.1, 162.1.

**GC-MS:** 221.9, 224.0

### Example-4: Preparation of Impurity (X)

The aqueous layer obtained in Example-3 was stirred for 16 h to obtain a precipitate. The precipitate was filtered, washed with water (50.0 ml) and dried under vacuum to obtain mixture of solid isomers i.e. *(E*/*Z)*-methyl-4,4-dichloro-2-((methylamino)methylene)-3-oxobutanoate (4.0 g, 8.2% yield, 17.70 mmol).

### Isomer-1 (Major):

**¹H NMR (CDCl_{3,} 400 MHz):** δ 10.86 (br s, 1H), 8.14(d, *J=* 14 Hz, 1H), 7.49 (s, 1H), 3.75 (s, 3H), 3.23 (d, *J* = 4.8 Hz, 3H).

**¹³C NMR (CDCl_{3,} 100 MHz):** δ 36.7, 37.0, 51.3, 51.4, 68.6, 163.5, 186.5.

### Isomer-2 (Minor):

**¹H NMR (CDCl_{3,} 400 MHz):** δ 9.38 (br s, 1H), 8.22 (d, *J=* 14.8 Hz, 1H), 7.11 (s, 1H), 3.82 (s, 3H), 3.23 (d, *J* = 4.8 Hz, 3H)

**¹³C NMR (CDCl_{3,} 100 MHz):** δ 36.6, 36.8, 51.2, 51.3, 51.5, 95.3, 165.95.

**LCMS:** 225.9 [M+1]

### Example-5: Preparation of methyl-3-(4-chlorophenyl)-1-methyl-1H-pyrazole-4-carboxylate

To a solution of 4-chlorobenzonitrile (1.0 g, 7.27 mmol) in toluene (12.0 ml) anhydrous copper(II) acetate (1.22 g, 6.72 mmol) was added, and the resulting mixture was heated to 50 °C for 1 h. To this reaction mixture, a solution of methyl-3-(methylamino)acrylate (0.82 g, 7.12 mmol) in toluene (12.0 ml) was added over 15 min at the same temperature. The reaction mixture was stirred for 20 h at 95 °C, cooled to 25 °C and filtered through a celite bed. The filtrate was washed with 10% aqueous citric acid solution (12.0 ml). The aqueous layer obtained by this procedure was extracted twice with ethyl acetate (56.0 ml). The combined organic layers were washed twice with water (40.0 ml), dried over anhydrous sodium sulphate and concentrated under reduced pressure to obtain methyl-3-(4-chlorophenyl)-1-methyl-1H-pyrazole-4-carboxylate (1.14 g, 62.6% yield, 4.55 mmol).

**¹H NMR (CDCl₃, 400 MHz):** δ 7.94 (s, 1H), 7.74 (d, *J* = 11.2 Hz, 2H), 7.37 (d, *J* =11.2 Hz, 2H), 3.94 (s, 3H), 3.77 (s, 3H)

**¹³C NMR (CDCl₃, 100 MHz):** δ 39.3, 51.2, 111.3, 128.0, 130.4, 130.7, 134.4, 135.8, 152.0, 163.2 **GC-MS:** 250.0, 252.0

### Example-6: Preparation of methyl 3-isopropyl-1-methyl-1H-pyrazole-4-carboxylate

To a solution of isobutyronitrile (1.0 g, 14.47 mmol) in toluene (12.0 ml) anhydrous copper(II) acetate (3.42 g, 18.83 mmol) was added, and the resulting mixture was heated to 50 °C for 1 h. To this reaction mixture, a solution of methyl-3-(methylamino)acrylate (1.81 g, 14.47 mmol) in toluene (12.0 ml) was added slowly. The reaction mixture was stirred for 20 h at 95 °C, cooled to 25 °C and filtered through a celite bed. The filtrate was washed with 10% aqueous citric acid solution (25.0 ml). The aqueous layer was extracted twice with ethyl acetate (56.0 ml). The combined organic layers were washed twice with water (40.0 ml), dried over anhydrous sodium sulphate and concentrated under reduced pressure to obtain methyl 3-vinyl-1H-pyrazole-4-carboxylate (0.73 g, 28% yield, 4.01 mmol).

**¹H NMR (CDCl_{3,}400 MHz):** δ 7.75 (s, 1H), 3.83 (s, 3H), 3.79 (s, 3H), 3.46-3.57 (m, 1H), 1.27 (s, 6H). **¹³C NMR (CDCl_{3,}100 MHz):** δ 21.8, 26.7, 38.9, 50.9, 110.4, 134.6, 160.6, 163.8.

**GC-MS:** 182.1

### Example-7: Preparation of methyl-1-methyl-3-vinyl-1H-pyrazole-4-carboxylate

To a solution of acrylonitrile (1.0 g, 18.85 mmol) in toluene (12.0 ml) was added anhydrous copper(II) acetate (3.48 g, 19.16 mmol) and heated to 50 °C for 1 h. To this reaction mixture, a solution of methyl-3-(methylamino)acrylate (2.36 g, 18.85 mmol) in toluene (12.0 ml) was added slowly over 15 min. Reaction mixture was stirred for 20 h at 95 °C, cooled to 25 °C and filtered through a celite bed. Filtrate was washed with 10% aqueous citric acid solution (33.0 ml). Aqueous layer was washed twice with ethyl acetate (56.0 ml). Combined organic layers were washed twice with water (40.0 ml), dried over anhydrous sodium sulphate and concentrated under reduced pressure to obtain methyl-1-methyl-3-vinyl-1H-pyrazole-4-carboxylate (1.10 g, 35.0% yield, 6.62 mmol).

¹H NMR (CDCl_{3,} 400 MHz): 8.01 (s, 1H), 7.40 (d, *J* = 4 Hz, 1H), 7.18 (d, *J=* 16.0 Hz, 1H), 6.00 (d, *J* = 12 Hz, 1H), 3.79 (s, 3H), 3.68 (s, 3H)

**GC-MS:** 166.1

### Example-8: Preparation of Impurity (IXA)

Tin(II) chloride dihydrate (1.960 g, 8.69 mmol) and toluene (12.0 ml) were mixed together followed by the addition of methyl-3-(methylamino)acrylate (1.042 g, 8.69 mmol) in toluene (12.0 ml) at 25 °C. The reaction mixture was stirred for 16 h at 70 °C. It was then cooled to 25 °C and filtered through a celite bed. The filtrate was washed with 6% aqueous NaHCO₃ solution (13.0 ml). The organic layer was washed twice with water (20.0 ml), dried over anhydrous sodium sulphate and concentrated under reduced pressure to obtain a crude solid. This crude solid was triturated with n-hexane (23.0 ml) and purified on combiflash chromatography (FCC) to obtain pure dimethyl-4-(2-methoxy-2-oxoethyl)-1-methyl-1,4-dihydropyridine-3,5-dicarboxylate (1.50 g, 61% yield, 8.70 mmol).

**¹H NMR (CDCl_{3,} 400 MHz):** δ 7.08 (s, 2H), 4.13 (t, *J=* 5.2 Hz, 1H), 3.69 (s, 6H), 3.55 (s, 3H), 3.14 (s, 3H), 2.41 (d, *J* = 5.2 Hz, 2H)

**¹³C NMR (CDCl_{3,} 100 MHz):** δ 29.0, 40.8, 41.3, 51.1, 51.2, 105.5, 140.1, 167.0, 171.8

**GCMS:** 252.1, 210.1, 150.1

**LCMS:** 284.0 [M+1], 210.0

### Example-9: Preparation of Impurity (VIA) and (VIIA)

To a solution of methyl-3-(methylamino)acrylate (5.0 g, 43.43 mmol) in toluene (115.0 ml) was added anhydrous copper(II) acetate (7.90 g, 43.43 mmol) at 25 °C. The reaction mixture was stirred for 16 h at 65-70 °C under vacuum (703 Torr) and then cooled to 25 °C. It was then filtered through a celite bed. The filtrate was washed with 10% aqueous citric acid solution (75.0 ml). The organic layer was dried over anhydrous sodium sulphate and concentrated under reduced pressure to obtain a mixture of Impurity (VIA) and VIIA. These components were separated on neutral alumina by flash column chromatography.

### Impurity VIA

**¹H NMR (CDCl_{3,} 400 MHz):** δ 8.77 (br s, 1H), 7.38 (d, *J=* 13.8 Hz, 1H), 7.17 (d, *J=* 12.2 Hz, 1H), 6.00 (d, *J* = 15.6 Hz, 1H), 3.76 (s, 3H), 3.71 (s, 3H), 3.10 (d, *J=* 4.8 Hz, 3H)

**LCMS:** 199.75 [M+1]

### Impurity VIIA

**¹H NMR (CDCl_{3,} 400 MHz):** δ 8.18 (d, *J=* 2.4 Hz, 1H), 7.84 (dd, *J¹* = 2.8 Hz, *J²* = 2.4 Hz, 1H), 6.52 (d, *J=* 4.8 Hz, 1H), 3.85 (s, 3H), 3.58 (s, 3H)

**LCMS:** 167.75 [M+1]

### Example-10: Preparation of Impurity (VIIIA)

Ethyl-2,2-difluoroacetate (150.0 g, 1209 mmol) was charged in ethyl alcohol (1500 ml) at 25 °C. The solution was cooled to 0-5 °C, and then ammonia gas was bubbled in slowly for 30 min. The reaction mixture was stirred for 2 h at 25 °C. Excess solvent was removed under reduced pressure to obtain the crude solid. The crude solid was triturated in n-hexane (458.0 ml) at 15-20 °C and filtered to obtain pure 2,2-difluoroacetamide (110.0, 96% yield, 1209 mmol).

**¹H NMR (CDCl_{3,} 400 MHz):** δ 6.37 (br s, 2H), 5.89 (t, *J=* 54 Hz, 1H)

**¹³C NMR (CDCl_{3,}100 MHz):** δ 165.1, 164.9, 164.6, 110.6, 108.1, 105.6

**GCMS:** 95.0, 44.0, 32.0

## Claims

1. A process for preparing substituted heterocyclic compound of Formula I or of salts thereof 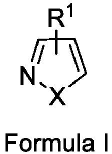
wherein **R¹** is selected from hydrogen, halogen, COOR³, C₁-C₆-alkyl, C₂-C₆-alkenyl, C₃-C₈-cyclohaloalkyl, C₁-C₆-haloalkyl, C₁-C₆-haloalkoxy, C₁-C₆-alkoxy-C₁-C₄-alkyl, C₃-C₈-cycloalkoxy, C₁-C₆-hydroxyalkyl, C₃-C₈-halocycloalkyl, C₁-C₆-haloalkoxycarbonyl, C₁-C₆-alkylthio, C₁-C₆-alkylsulfinyl, C₁-C₆-alkylsulfonyl, C₁-C₆-haloalkylthio, C₁-C₆-haloalkylsulfinyl, C₁-C₆-haloalkylsulfonyl, C₃-C₈-cycloalkylthio, C₁-C₆-alkylamino, C₁-C₆-dialkylamino, C₃-C₈-cycloalkylamino, C₃-C₈-cycloalkyl-C₁-C₆-alkylamino, C₁-C₆-alkoxycarbonyl, C₁-C₆-alkoxycarbonyloxy, C₁-C₆-alkylaminocarbonyloxy, C₁-C₆-dialkylaminocarbonyloxy, 5- to 11-membered spirocyclic ring or 3- to 10- membered carbocyclic ring; wherein Formula I may be substituted by one or more R¹;
**X** is NR²;
**R²** is C₁-C₆-alkyl;
**R³** is C₁-C₆-alkyl,
which comprises reacting a compound of Formula II or of salts thereof R¹ 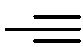 N
**Formula II**
wherein R¹ has the same meaning as defined above; with a compound of Formula III or of salts thereof 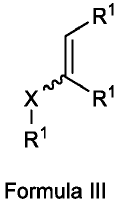
wherein X and R¹ have the same meaning as defined above;
in the presence of a catalyst and optionally in the presence of a solvent selected from one or more of aliphatic, alicyclic or aromatic hydrocarbons and ethers; wherein said reaction is carried out at temperature in the range of 50°C to 95°C.

2. The process as claimed in claim 1, wherein the catalyst is selected from one or more of copper powder, anhydrous copper(II) acetate, copper(II) acetate monohydrate, copper(I) oxide, copper(II) oxide, copper(I) chloride, copper(II) chloride, iron(II) chloride, iron(III) chloride, zinc(II) chloride, nickel(II) acetate, cobalt(II) chloride, cobalt(II) chloride hexahydrate, cobalt(II) bromide, cobalt(II) fluoride, cobalt(II) iodide, cobalt(II) oxide, cobalt(III) oxide, cobalt(II, III) oxide, cobalt(II) acetate anhydrous and cobalt(II) acetate tetrahydrate.

3. The process as claimed in claim 1, wherein said reaction is carried out at temperature in the range of 70 °C to 90 °C.

4. A process as claimed in claim 1 for preparing methyl-3-(dihalomethyl)-1-methyl-1H-pyrazole-4-carboxylate compound of Formula 1A or of salts thereof 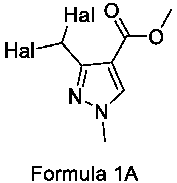 comprising reacting 2,2-dihaloacetonitrile with methyl-3-(methylamino)acrylate in the presence of a catalyst and optionally in the presence of a solvent selected from one or more of aliphatic, alicyclic or aromatic hydrocarbons and ethers.

5. The process as claimed in claim 4, wherein the catalyst is selected from one or more of copper powder, anhydrous copper(II) acetate, copper(II) acetate monohydrate, copper(I) oxide, copper(II) oxide, copper(I) chloride, copper(II) chloride, iron(II) chloride, iron(III) chloride, zinc(II) chloride, nickel(II) acetate, cobalt(II) chloride, cobalt(II) chloride hexahydrate, cobalt(II) bromide, cobalt(II) fluoride, cobalt(II) iodide, cobalt(II) oxide, cobalt(III) oxide, cobalt(II, III) oxide, cobalt(II) acetate anhydrous and cobalt(II) acetate tetrahydrate.

## Patentansprüche

1. Verfahren zur Herstellung von substituierter heterocyclischer Verbindung der Formel I oder von Salzen davon: 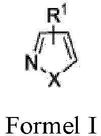
wobei R¹ ausgewählt ist aus Wasserstoff, Halogen, COOR³, C₁-C₆-Alkyl, C₂-C₆-Alkenyl, C₃-C₈-Cyclohalogenalkyl, C₁-C₆-Halogenalkyl, C₁-C₆-Halogenalkoxy, C₁-C₆-Alkoxy-C₁-C₄-alkyl, C₃-C₈-Cycloalkoxy, C₁-C₆-Hydroxyalkyl, C₃-C₈-Halogencyclo-alkyl, C₁-C₆-Halogenalkoxycarbonyl, C₁-C₆-Alkylthio, C₁-C₆-Alkylsulfinyl, C₁-C₆-Alkylsulfonyl, Ci-Ce-Halogenalkylthio, C₁-C₆-Halogenalkylsulfinyl, C₁-C₆-Halogenalkylsulfonyl, Cs-Cs-Cycloalkylthio, C₁-C₆-Alkylamino, C₁-C₆-Dialkylamino, C₃-C₈-Cycloalkylamino, C₃-Cs-Cycloalkyl-C₁-C₆-alkylamino, C₁-C₆-Alkoxycarbonyl, Ci-C₆-Alkoxycarbonyloxy, C₁-C₆-Alkylaminocarbonyloxy, Ci-Ce-Dialkylaminocarbonyloxy, 5- bis 11-gliedrigem spirocyclischem Ring oder 3- bis 10-gliedrigem carbocyclischem Ring; wobei Formel I mit einem oder mehreren R¹ substituiert sein kann;
X NR² ist;
R² C₁-C₆-Alkyl ist;
R³ C₁-C₆-Alkyl ist;
welches Umsetzen einer Verbindung der Formel II oder Salzen davon: R¹ 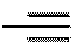 N
Formel II
wobei R¹ die gleiche Bedeutung wie oben definiert hat;
mit einer Verbindung der Formel III oder Salzen davon: 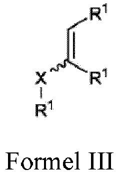
wobei X und R¹ die gleiche Bedeutung wie oben definiert haben;
in Gegenwart eines Katalysators und gegebenenfalls in Gegenwart eines Lösungsmittels ausgewählt aus einem oder mehreren von aliphatischen, alicyclischen oder aromatischen Kohlenwasserstoffen und Ethern umfasst; wobei die Reaktion bei einer Temperatur im Bereich von 50 °C bis 95 °C durchgeführt wird.

2. Verfahren nach Anspruch 1, wobei der Katalysator ausgewählt ist aus einem oder mehreren von Kupferpulver, wasserfreiem Kupfer(II)acetat, Kupfer(II)acetat-monohydrat, Kupfer(I)oxid, Kupfer(II)oxid, Kupfer(I)chlorid, Kupfer(II)chlorid, Eisen(II)-chlorid, Eisen(III)chlorid, Zink(II)chlorid, Nickel(II)acetat, Kobalt(II)chlorid, Kobalt(II)chlorid-hexahydrat, Kobalt(II)bromid, Kobalt(II)fluorid, Kobalt(II)iodid, Kobalt(II)oxid, Kobalt(III)oxid, Kobalt(II, III)oxid, Kobalt(II)acetat wasserfrei und Kobalt(II)acetat-tetrahydrat.

3. Verfahren nach Anspruch 1, wobei die Reaktion bei einer Temperatur im Bereich von 70 °C bis 90 °C durchgeführt wird.

4. Verfahren nach Anspruch 1 zur Herstellung von Methyl-3-(dihalogenmethyl)-1-methyl-1H-pyrazol-4-carboxylatverbindung der Formel 1A oder Salzen davon: 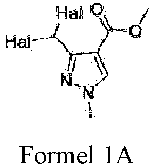 umfassend Umsetzen von 2,2-Dihalogenacetonitril mit Methyl-3-(methylamino)-acrylat in Gegenwart eines Katalysators und gegebenenfalls in Gegenwart eines Lösungsmittels ausgewählt aus einem oder mehreren von aliphatischen, alicyclischen oder aromatischen Kohlenwasserstoffen und Ethern.

5. Verfahren nach Anspruch 4, wobei der Katalysator ausgewählt ist aus einem oder mehreren von Kupferpulver, wasserfreiem Kupfer(II)acetat, Kupfer(II)acetat-monohydrat, Kupfer(I)oxid, Kupfer(II)oxid, Kupfer(I)chlorid, Kupfer(II)chlorid, Eisen(II)-chlorid, Eisen(III)chlorid, Zink(II)chlorid, Nickel(II)acetat, Kobalt(II)chlorid, Kobalt(II)chlorid-hexahydrat, Kobalt(II)bromid, Kobalt(II)fluorid, Kobalt(II)iodid, Kobalt(II)oxid, Kobalt(III)oxid, Kobalt(II, III)oxid, Kobalt(II)acetat wasserfrei und Kobalt(II)acetat-tetrahydrat.

## Revendications

1. Procédé de préparation d'un composé hétérocyclique substitué de formule I ou de sels de celui-ci 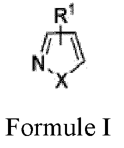
où R¹ est choisi parmi hydrogène, halogène, COOR³, alkyle en C₁-C₆, alcényle en C₂-C₆, cyclohalogénoalkyle en C₃-C₈, halogénoalkyle en C₁-C₆, halogénoalcoxy en C₁-C₆, alcoxy en C₁-C₆-alkyle en C₁-C₄, cycloalcoxy en C₃-C₈, hydroxyalkyle en Ci-C₆, halogénocycloalkyle en C₃-C₈, halogénoalcoxycarbonyle en C₁-C₆, alkylthio en C₁-C₆, alkylsulfinyle en C₁-C₆, alkylsulfonyle en C₁-C₆, halogénoalkylthio en C₁-C₆, halogénoalkylsulfinyle en C₁-C₆, halogénoalkylsulfonyle en C₁-C₆, cycloalkylthio en C₃-C₈, alkylamino en C₁-C₆, dialkylamino en C₁-C₆, cycloalkylamino en C₃-C₈, cycloalkyl en C₃-C₈-alkylamino en C₁-C₆, alcoxycarbonyle en C₁-C₆, alcoxycarbonyloxy en C₁-C₆, alkylaminocarbonyloxy en C₁-C₆, dialkylaminocarbonyloxy en C₁-C₆, un cycle spirocyclique à 5 à 11 chaînons ou un cycle carbocyclique à 3 à 10 chaînons ; dans lequel la formule I peut être substituée par un ou plusieurs R¹ ;
X est NR² ;
R² est alkyle en C₁-C₆ ;
R³ est alkyle en C₁-C₆,
qui comprend la réaction d'un composé de formule II ou de sels de celui-ci R¹ 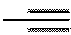 N
Formule II
où R¹ a la même signification que définie ci-dessus ;
avec un composé de formule III ou de sels de celui-ci 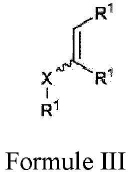
où X et R¹ ont la même signification que définie ci-dessus ;
en présence d'un catalyseur et éventuellement en présence d'un solvant choisi parmi un ou plusieurs parmi les hydrocarbures et éthers aliphatiques, alicycliques ou aromatiques ; dans lequel ladite réaction est effectuée à une température dans la plage de 50 °C à 95 °C.

2. Procédé selon la revendication 1, dans lequel le catalyseur est choisi parmi un ou plusieurs parmi une poudre de cuivre, l'acétate de cuivre(II) anhydre, l'acétate de cuivre(II) monohydraté, l'oxyde de cuivre(I), l'oxyde de cuivre(II), le chlorure de cuivre(I), le chlorure de cuivre(II), le chlorure de fer(II), le chlorure de fer(III), le chlorure de zinc(II), l'acétate de nickel(II), le chlorure de cobalt(II), le chlorure de cobalt(II) hexahydraté, le bromure de cobalt(II), le fluorure de cobalt(II), l'iodure de cobalt(II), l'oxyde de cobalt(II), l'oxyde de cobalt(III), l'oxyde de cobalt(II, III), l'acétate de cobalt(II) anhydre et l'acétate de cobalt(II) tétrahydraté.

3. Procédé selon la revendication 1, dans lequel ladite réaction est effectuée à une température dans la plage de 70 °C à 90 °C.

4. Procédé selon la revendication 1 de préparation d'un composé 3-(dihalogénométhyl)-1-méthyl-1H-pyrazole-4-carboxylate de méthyle de formule 1A ou de sels de celui-ci 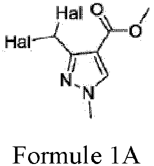 comprenant la réaction de 2,2-dihalogénoacétonitrile avec du 3-(méthylamino)acrylate de méthyle en présence d'un catalyseur et éventuellement en présence d'un solvant choisi parmi un ou plusieurs parmi les hydrocarbures et éthers aliphatiques, alicycliques ou aromatiques.

5. Procédé selon la revendication 4, dans lequel le catalyseur est choisi parmi un ou plusieurs parmi une poudre de cuivre, l'acétate de cuivre(II) anhydre, l'acétate de cuivre(II) monohydraté, l'oxyde de cuivre(I), l'oxyde de cuivre(II), le chlorure de cuivre(I), le chlorure de cuivre(II), le chlorure de fer(II), le chlorure de fer(III), le chlorure de zinc(II), l'acétate de nickel(II), le chlorure de cobalt(II), le chlorure de cobalt(II) hexahydraté, le bromure de cobalt(II), le fluorure de cobalt(II), l'iodure de cobalt(II), l'oxyde de cobalt(II), l'oxyde de cobalt(III), l'oxyde de cobalt(II, III), l'acétate de cobalt(II) anhydre et l'acétate de cobalt(II) tétrahydraté.
